# EUROPEAN PATENT SPECIFICATION

(11) **EP 2 709 599 B1**
(45) Date of publication and mention of the grant of the patent: **20.12.2017**
(21) Application number: 12721534.1
(22) Date of filing: 16.05.2012
(51) Int. Cl.: A61K 9/48, A61K 31/47, A61K 31/485

(54) **PHARMACEUTICAL COMPOSITION COMPRISING DROTAVERINE**
PHARMAZEUTISCHE ZUSAMMENSETZUNG MIT DROTAVERIN
COMPOSITION PHARMACEUTIQUE CONTENANT DE LA DROTAVÉRINE

(30) Priority: 20.05.2011 IN 1728CH2011; 22.06.2011 US 201161499850 P; 13.07.2011 EP 11305922
(43) Date of publication of application: 26.03.2014
(73) Proprietor: Chinoin Private Co Ltd, 1045 Budapest (HU)
(72) Inventor: BADABHAGNI, Sudhakara Rao, 75008 Paris (FR); JAISWAL, Nilesh, 75008 Paris (FR); KHULLAR, Praveen, 75013 Paris (FR); PRASAD, Kum, 75008 Paris (FR)
(74) Representative: Taravella, Brigitte
(86) International application number: PCT/EP2012/059163
(87) International publication number: WO 2012/159964

(56) References cited:
- EP-A1- 0 513 997
- US-A- 4 814 336
- US-A1- 2003 077 297
- BOLAJI ET AL.: "Pharmacokinetics and bioavailability of drotaverine in humans", EUROPEAN JOURNAL OF DRUG METABOLISM AND PHARMACOKINETICS, vol. 21, no. 3, 1996, pages 217-221, XP002665739, cited in the application
- DYDERSKI ET AL: "Bioavailability study of drotaverine from capsule and tablet preparation in healthy volunteers", ARZNEIMITTEL FORSCHUNG. DRUG RESEARCH, ECV EDITIO CANTOR VERLAG, AULENDORF, DE, vol. 54, no. 5, 1 January 2004 (2004-01-01), pages 298-302, XP008145497, ISSN: 0004-4172

## Description

### FIELD OF THE INVENTION:

The present invention relates to a stable pharmaceutical composition of drotaverine hydrochloride for oral administration. The present invention pertains a pharmaceutical composition in the form of a soft capsule for oral administration comprising:
- 5 to 30% (w/w) of drotaverine hydrochloride;
- at least 60% (w/w) of a liquid mixture of a non-ionic hydrophilic surfactant and a non-ionic hydrophobic surfactant, wherein the non-ionic hydrophilic surfactant is polysorbate 80 and the non-ionic hydrophobic surfactant is propylene glycol monocaprylate;
- wherein the weight ratio of drotaverine hydrochloride to the liquid mixture of surfactant is from 1:3 to 1:7.

The present invention furthermore also relates to a process for the preparation of such pharmaceutical composition and the use of said composition for the preparation of a drug product for treating spasms and acute pains.

### BACKGROUND OF THE INVENTION:

Drotaverine (1-(3,4-diethoxybenzylidene)-6,7-diethoxy-1,2,3,4-tetrahydroisoquinoine) belongs to the class of drug known as hydroisoquinolone of the following formula:

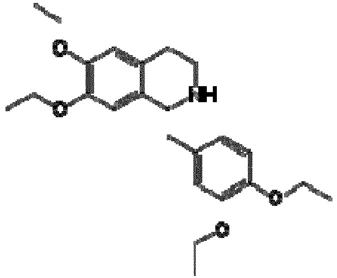

It belongs to cholinergic muscarinic antagonist pharmacological group on the basis of mechanism of action and also classified in Antispasmodic Agent pharmacological group. The Molecular Weight of drotaverine is 397.50. Drotaverine is considered to have smooth muscle relaxant properties. Their effect depended upon the concentration applied. It is a non-anticholinergic antispasmodic, which selectively inhibits phosphodiesterase IV and is accompanied by a mild calcium channel-blocking effect. It is commercialized in tablet form under the trademark No-spa® mainly in some countries of Eastern and Central Europe and Sub Saharan Africa. Drotaverine is primarily indicated in conditions like cholangitis, cholecystitis, cholecytolithiasis, cystitis, nephrolithiasis, papilitis, smooth muscle spasm, stone formation, ureterolithiasis, urolithiasis, vesical tenesmus.

A difficulty in the formulation of drotaverine in oral pharmaceutical compositions is its unpleasant, strong and bitter taste and after taste which has led to poor, or even non-compliance with the treatment and thus has a negative impact on the efficiency of treatment.

Furthermore, pharmacokinetics and bioavailability studies conducted for drotaverine hydrochloride oral dose on healthy individuals indicated that the absolute bioavailability was variable and ranged from 24.5% to 91% with a mean of 58.2 to 18.2% (mean SD) (European Journal of Drug Metabolism and Pharmacokinetics vol: 21, Issue: 3, Pages: 217-221). It is suggested that the high variation in the bioavailability of drotaverine hydrochloride after oral administration may result in significant inter-individual differences in therapeutic response.

Several documents disclose drotaverine oral dosage forms, for example EP0513997 A1 and US4814336 A.

The object of the present invention is to provide preparations such as a soft capsule which allows obtain pharmacokinetic parameters bioequivalent to those which are obtained with the drotaverine hydrochloride tablets in a substantially equivalent dose such available under the trademark No-spa®.

Another aspect of the invention is to provide a method for preparing the oral pharmaceutical composition of the invention, comprising dissolving a drug in an appropriate amount of a liquid carrier and bringing the pH to an acceptable range whereby the storage stability and the shelf-life of the formulation is obtained.

Another object of the invention is to provide an oral formulation of the drotaverine hydrochloride with a reduced bitter taste.

Applicants have found that theses objects can be achieved by providing a liquid composition comprising drotaverine hydrochloride in a liquid mixture of at least one non-ionic hydrophilic surfactant and at least one non-ionic hydrophobic surfactant.

### SUMMARY

The present invention provides a pharmaceutical composition of drotaverine hydrochloride for oral administration comprising drotaverine hydrochloride and a liquid mixture of at least one non-ionic hydrophilic surfactant and at least one non-ionic hydrophobic surfactant; the pharmaceutical composition may optionally contain suitable pharmaceutically acceptable excipients.

It is yet another object of the present invention to provide a pharmaceutical composition, for instance, a soft capsule, a hard capsule, two-piece capsule or tablet comprising the composition of the instant invention.

In another aspect of the invention there is provided a process for the preparation of the pharmaceutical composition of the invention, comprising dissolving a drug in an appropriate amount of solvent system.

The invention also relates to the use of the oral pharmaceutical composition of the invention for the preparation of a drug for the treatment of spasms and acute pains.

These and other aspects, features and advantages of the present invention will become better understood with reference to the following description and claims.

### DESCRIPTION

Several experiments were carried out by the present inventors in order to achieve a liquid pharmaceutical composition of drotaverine having a bioavailability comparable to that obtained for the NoSpa® tablet, wherein it was surprisingly observed that drotaverine in solvent system played a vital role in oral bioavailability. It was found that drotaverine in a liquid mixture of at least one non-ionic hydrophilic surfactant and at least one non-ionic hydrophobic surfactant allow to obtain a bioavailability which is equivalent to that obtained for the NoSpa® tablet.

"Bioequivalent" as employed herein means that if the pharmaceutical composition according to the invention is tested in a study by comparison with a reference marketed product, the average Area under the Curve (AUC) and/or the Cmax for each drug product is at least 80% of the (corresponding) mean AUC and/or Cmax observed.

According to the invention, a stable formulation means a formulation which, in particular, exhibits high resistance against decomposition of drotaverine hydrochloride. Thus, upon storage during 1 month at 40 deg. C. and 75% humidity, the pharmaceutical composition according to the present invention contains at least 99% (w/w) of the initial drotaverine hydrochloride and does not exhibit any sign of high level of decomposition, i.e. the level of total impurities is less than 1% by weight based on the drotaverine hydrochloride (as evidenced by HPLC analysis).

The drotaverine hydrochloride is taken in therapeutically effective amounts. "Therapeutically effective amount" should be understood as meaning a dose of the drug effective in exerting a therapeutic effect. For an oral preparation of the invention, the term "therapeutically effective amount" means a dose of the drug which, after absorption into the body through the walls of gastrointestinal tract, yields a drug concentration in the blood effective in exerting a therapeutic effect on a target organ. Persons of ordinary skill in the art will understand that the amounts of the drug presented in the composition vary with the particular situation, including but not limited to, the mode of administration, the size, age and condition of the subject and the like. Moreover, these effective amounts can be easily determined by the physician without undue experimentation.

In the composition of the present invention, drotaverine hydrochloride is present in amounts ranging from 5% to 30% by weight of the composition. In a preferred embodiment the drotaverine hydrochloride is present in amounts ranging 10% to 30% by weight of the composition. The pharmaceutical composition is in the form of a soft capsule for oral administration comprising:
- 5 to 30% (w/w) of drotaverine hydrochloride;
- at least 60% (w/w) of a liquid mixture of a non-ionic hydrophilic surfactant and a non-ionic hydrophobic surfactant, wherein the non-ionic hydrophilic surfactant is polysorbate 80 and the non-ionic hydrophobic surfactant is propylene glycol monocaprylate;
- wherein the weight ratio of drotaverine hydrochloride to the liquid mixture of surfactant is from 1:3 to 1:7.

The total amount of surfactant is at least of 60%, and preferably from 60 to 95% by weight, based on the total weight of the composition. More preferably, the total amount of surfactant is from 75 to 90% by weight of the composition. The non-ionic hydrophobic surfactant employed in context of the present invention is propylene glycol monocaprylate (capryol-90) which has an HLB value of 6.

In the composition of the present invention, it is preferred that the hydrophobic surfactant is present in amounts ranging from 60% to 90% by weight of the composition. More preferably, the hydrophobic surfactant is present in amounts ranging from 80% to 85% by weight of the composition.

The hydrophilic surfactant for including in the pharmaceutical composition of the present invention is polysorbate 80 (polyoxyethylene sorbitan monooleate; Tween 80) which has an HLB value of 15.

In the composition of the present invention, it is preferred that the hydrophilic surfactant is present in amounts ranging from 1% to 10% by weight of the composition. Most preferably, the hydrophilic surfactant is present in amounts ranging from 3% to 7% by weight of the composition.

In a most preferred embodiment of the invention, the pharmaceutical composition is a mixture of propylene glycol monocaprylate (capryol-90) (the non-ionic hydrophobic surfactant) and polysorbate 80 (the non-ionic hydrophilic surfactant).

The drotaverine composition according to the present invention may further comprise a viscosity modifier and optionally a preservative.

The viscosity modifiers used in the present invention may be any of those known in the art such as cellulosic derivatives, swellable polymers, gums, polyoxyethylene copolymers and likewise.

The preservatives used in the present invention may be any of those known in the art such as sodium metabisulphite, sodium sulphite, sodium benzoate, benzoic acid, di sodium EDTA, alpha tocopherol, propyl gallate, butylated hydroxyl anisole and butylated hydroxyl toluene, ascorbic acid and likewise.

In a preferred embodiment, the pH of the solvent system is maintained from 4 to 6. More particularly, the pH is in the range of 4.5-5.8. Most preferably, the pH is maintained at 4.5. The pH of the solvent system can be adjusted using any conventional buffer. The preferred buffer used in acetate buffer.

The invention also relates to a method for preparing a pharmaceutical preparation comprising 5% to 30% by weight of the composition of drotaverine hydrochloride and from 60% to 95% by weight of the composition of a liquid mixture of the non-ionic hydrophilic surfactant and the non-ionic hydrophobic surfactant. This method comprises the following steps: dissolving the drotaverine hydrochloride in a liquid mixture of the non-ionic hydrophilic surfactant and the non-ionic hydrophobic surfactant, with stirring, in order to obtain an homogeneous mixture; and then adjust the pH from 4 to 6 using a conventional buffer.

One aspect of the invention provides for soft gelatin capsules which include a capsule shell comprising gelatin and/or plasticizers and, if desired or required, further auxiliary materials.

In developing the soft gelatin capsule for drotaverine composition according to the present invention, it must be recognized that the capsule is a system comprised of the drotaverine composition and the gelatin shell used to encapsulate the drotaverine composition. As such, not only is the filled drotaverine composition preferred to produce the desired oral bioavailability characteristics but the gelatin shell composition is also preferred as it must be compatible with the drotaverine composition. One skilled in the art would be aware of the potential fill-shell interactions which could result in both physical and chemical capsule instability. Accordingly, the gelatin shell composition utilized to form the capsule for the drotaverine composition is also preferred and is significant to the present invention.

In general, gelatin shell capsule composition for soft gelatin capsules consist of raw gelatin and one or more ingredients which are added to plasticize the gelatin to produce a capsule to suitable hardness as required by design or by preference. Typical plasticizers include glycerin and sorbitol. Also, sorbitan anhydrides and mannitol may also be utilized. Furthermore, other non-traditional ingredients may also be used to plasticize the gelatin.

A major problem with gelatin-based composition is an apparent fall in dissolution upon aging, which is attributed to the cross-linking of gelatin-containing products. The cross-linking causes the formation of a swollen, very thin, tough, rubbery, water-insoluble membrane, also known as pellicle. The pellicle acts as a barrier and restricts the release of the drug. Drugs like drotaverine or its pharmaceutically acceptable salts or hydrates thereof, have tendency to react with gelatin and induce cross linking owing to which the possibility of fall in dissolution during stability studies is high. The present inventors have found that such cross linking of gelatin is surprisingly overcome by addition of certain weak acids in combination with glycine. In a particular embodiment of the invention, the weak acid employed in the present invention is tartaric acid, citric acid or its combinations thereof. The weak acid may be present in an amount of about 0.1 to 1.0% on wt basis of the gelatin shall formula.

As such, the gelatin compositions suitable for use with the drotaverine composition of the present invention provide the necessary physical and chemical stability required.

The preferred gelatin composition for use in constructing soft gelatin capsules for use with the drotaverine composition of the present invention includes gelatin and a plasticizer. Such plasticizer, which are well known in the pharmaceutical composition art, include, for example, propylene glycol, and sorbitol. Suitable plasticizers for use with the preferred capsule composition include sorbitol such as the Special™ MDF 85 from SPI Pharma.

The capsule compositions can also include other suitable additives such as preservatives and/or coloring agents which are utilized to stabilize the capsule and/or impart a specific characteristic such as color or look to the capsule. Pharmaceutically acceptable preservatives can include, for example, methyl and propyl parabens. Color may be imparted to the gelatin shell using FD&C and/or D&C dyes. Exemplary dyes include but are not limited to Tartrazine yellow, Azura red and the like. Opacifiers, such as titanium dioxide and/or iron oxides, may be employed to color and/or render the capsule opaque.

The invention contemplates use of coating agents which may include both non-functional or enteric coating agents such as cellulose based polymers, film coating agents or other coating agents known to a person of skilled in the art.

The present invention also contemplates the use of other pharmaceutical excipients such as buffering agents, alkalizers, acidifiers, binders, disintegrants, diluents, lubricants, plasticizers, permeation enhancers and solubilizers known to a person skilled in the art.

Other additives conventionally used in pharmaceutical compositions can be included, and these additives are well known in the art. Such additives include antioxidants such as Butylhydroxytoluene (BHT), preservatives, chelating agents, complexing agents, viscomodulators, tonicifiers, flavorants, colorants odorants, opacifiers, suspending agents, binders, and mixtures thereof. The amounts of such additives can be readily determined by one skilled in the art, according to the particular properties desired.

The exact dose of active agent and the particular composition to be administered depend on a number of factors, e.g., the condition to be treated, the desired duration of the treatment and the rate of release of the active agent. For instance, the amount of the active agent required and the release rate thereof may be determined on the basis of known in vitro or in vivo techniques, determining how long a particular active agent concentration in the blood plasma remains at an acceptable level for a therapeutic effect.

As soon as the basic principles for realization of the pharmaceutical compositions according to the present invention in the form of soft gelatin capsules are understood, the expert of pharmaceutical compositions will have no difficulty at all in adapting the process criteria to the particular needs.

Still another aspect of the invention is to provide a method of administering a pharmaceutical active ingredient to a subject taking into account the variability in bioavailability of the pharmaceutical active agent drotaverine which comprises the steps of: a) providing a stable gelatin composition of the invention for oral administration; and b) administering said composition to said host for ingestion.

In a further embodiment, the active agent comprises about 40 to 80 mg of the core composition.

The core ingredients of a typical composition according to the present invention may comprise:
- 5% to 30% (W/W) of drotaverine hydrochloride,
- At least 60% (w/w) of a liquid mixture of a non-ionic hydrophilic surfactant which is polysorbate 80 and a non-ionic hydrophobic surfactant which is propylene glycol monocaprylate.
The buffering agent used in the present invention may be any of those known in the art such as acetic acid, phosphoric acid, potassium phosphate, oxalic acid, carbonic acid, sodium carbonate, sodium acetate, sodium citrate, lithium oxalate, ammonium hydroxide, ammonium nitrate, citric acid, sodium bicarbonate, glycine, etc and/or their combinations.

The alkalizer used in the present invention may be selected from calcium carbonate, magnesium hydroxide, gum acacia, dicalcium phosphate, potassium hydroxide, ascorbic acid, etc and/or their combinations.

The acidifier used in the present invention may be selected from lactic acid, ascorbic acid, citric acid, phosphoric acid, calcium chloride etc and/or their combinations.

The gelatin shell ingredients of a typical composition according to the present invention may comprise:
- 35-52 % (w/w), more preferably 40-44% of gelatin,
- 15-35 % (w/w), more preferably 15-25% of sorbitol and/or glycerine,
- 0.1-10 % (w/w) of coloring agents,
- 0.1-10 % (w/w) of tartaric acid.

In another preferred embodiment the active filling (core composition) of the invention is encapsulated in a soft gelatin capsule of round/oval shape.

In a further aspect of the invention, there is provided a process for the preparation of the pharmaceutical composition. The process comprises incorporating the active ingredient drotaverine or its pharmaceutically acceptable salt or hydrates thereof into the lipophilic and/or hydrophilic carrier. Additionally, if appropriate, suitable pharmaceutical excipients are added to formulate an emulsion of the drug. In a preferred embodiment, a suitable carrier is continuously stirred with the active ingredient. The excipients were subsequently mixed.

Soft gelatin capsules are manufactured using rotary die process utilizing gelatin in a conventional process. Dry gelatin granules are combined with water and suitable plasticizers and the combination is then mixed and heated under vacuum to forma molten gelatin mass. The gelatin mass is held in its molten stage while being formed or cast into films or ribbons on casting wheels or drums. The films or ribbons are fed under the wedge and between rotary encapsulation dies. Within the encapsulation dies, capsules are simultaneously formed in pockets in the dies from the films or ribbons. The composition containing drotaverine is filled into the soft gelatin capsules using any conventional method. The capsule is then cut and sealed. The seals are formed via a combination of pressure and heat as the capsule is filled and cut.

The present invention is further defined in the following Examples. It should be understood that these examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various uses and conditions.

### EXAMPLE 1: Composition of drotaverine soft gelatin capsules (80 mg)

| **Ingredients** | **Function** | **mg/caps** | **(%w/w)** |
|---|---|---|---|
| drotaverine hydrochloride | active ingredient | 80.0 | 16.0 |
| propyleneglycol monocaprylate (Caproyl® 90) | hydrophobic surfactant | 409.75 | 82.0 |
| polysorabate 80 (Tween® 80) | hydrophilic surfactant | 10.0 | 2.0 |
| butylhydroxytoluene (BHT) | antioxidant | 0.25 | 0.05 |
| Total fill wt. | | 500 mg | |
| gelatin (Bloom : 150) | plasticizer | 259.1 | 60.26 |
| sorbitol special MDF 85 | plasticizer | 103.2 | 24.00 |
| coloring agent | coloring agent | 2.2 | 0.51 |
| tartaric acid | cross linking of gelatin inhibitor | 3.2 | 0.74 |
| Shell mass wt. (mg) | | 430 | |

### EXAMPLE 2: Composition of drotaverine soft gelatin capsules (40 mg)

| **Ingredients** | **Function** | **mg/caps** | **(%w/w)** |
|---|---|---|---|
| drotaverine hydrochloride | active ingredient | 40.0 | 16.0 |
| propyleneglycol monocaprylate (Caproyl® 90) | hydrophobic surfactant | 204.875 | 82.0 |
| polysorabate 80 (Tween® 80) | hydrophilic surfactant | 5.0 | 2.0 |
| butylhydroxytoluene (BHT) | antioxidant | 0.125 | 0.05 |
| Total fill wt. | | 250 mg | |
| gelatin (Bloom : 150) | plasticizer | 259.1 | 60.26 |
| sorbitol special MDF 85 | plasticizer | 103.2 | 24.00 |
| coloring agent | coloring agent | 2.2 | 0.51 |
| tartaric acid | cross linking of gelatin inhibitor | 3.2 | 0.74 |
| Shell mass wt. (mg) | | 430 | |

### EXAMPLE 3: Comparative in vitro dissolution and in vivo bioavailability studies between the drotaverine tablet (No-spa®) and a pharmaceutical composition according to the invention

The composition of drotaverine soft gelatin capsules used during the tests is the following:

| **Ingredients** | **Function** | **mg/caps** | **(%w/w)** |
|---|---|---|---|
| drotaverine hydrochloride | active ingredient | 80.0 | 16.0 |
| propyleneglycol monocaprylate (Caproyl® 90) | hydrophobic surfactant | 394.75 | 78.95 |
| polysorabate 80 (Tween® 80) | hydrophilic surfactant | 25.0 | 5.0 |
| butylhydroxytoluene (BHT) | antioxidant | 0.25 | 0.05 |
| Total fill wt. | | 500 mg | |
| gelatin (Bloom : 150) | Gelatin as diluent | 259.1 | 60.26 |
| sorbitol special MDF 85 | plasticizer | 103.2 | 24.00 |
| coloring agent | coloring agent | 2.2 | 0.51 |
| Shell mass wt. (mg) | | 430 | |

### Methodology:

### a) Test solution:

Weigh and drop 1 capsule in each of six dissolution vessels which contains dissolution medium. After specified time point withdraw 10 ml of the aliquot from a zone midway between the surface of the dissolution medium and the top of the rotating blade, not less than 10 mm away from the wall of the vessel. Filter the solution through 0.45 µm nylon 66 filter paper.

### b) Chromatographic conditions:

| | |
|---|---|
| Equipment | : Liquid chromatograph equipped |
| | withUV detector |
| Column | : X' terra, C18, 150 mm x 4.6 mm, 5 µm or equivalent |
| Column temperature | : 25°C |
| Flow rate | : 1.2 ml/min |
| Detection wavelength | : 240 nm |
| Injection volume | : 20 µl |
| Run time | : 6 min |

### c) Procedure:

Inject diluent as blank, five replicates of standard solution and single injection of each test solution into the chromatographic system, record the chromatograms and measure the peak responses of major peak.

### d) Dissolution Media Composition

| Ingredients of Media | Conc. |
|---|---|
| Sodium taurocholate (µM) | 80 |
| Lecithin (µM) | 20 |
| Pepsin (mg/ml) | 0.1 |
| Sodium chloride (mM) | 34.2 |
| Hydrochloric acid pH 1.6 | qs |
| Deionized water pH 1.6 | 1 L |
| Osmolality (mOsm kg-1) | 120.7±2.5 |

### e) Dissolution Conditions

- Apparatus: basket
- Volume: 500 ml
- Media : FaSGF: Fasted Simulated gastric Fluid-pH 1.6 Speed : 100 rpm

### Results:

### Dissolution profiles:

| | % drug release in pH 1.6 FaSGF / 100 rpm/Basket /500 ml | | | | |
|---|---|---|---|---|---|
| | **No Spa Tablets 80 mg** | | | | |
| **Tablet No**. | **15mins** | **30mins** | **45mins** | **60mins** | **Infinity mins** |
| **1** | 98 | 100 | 101 | 101 | 102 |
| **2** | 95 | 99 | 100 | 100 | 102 |
| **3** | 97 | 102 | 103 | 103 | 104 |
| Mean | 97 | 100 | 101 | 101 | 103 |
| Minimum | 95 | 99 | 100 | 100 | 102 |
| Maximum | 98 | 102 | 103 | 103 | 104 |
| %RSD | 1.6 | 1.5 | 1.5 | 1.5 | 1.1 |

| | % drug dissolved in pH 1.6 FaSGF / 100 rpm/Basket /500 ml | | | | |
|---|---|---|---|---|---|
| | **DROTAVERINE HCL Soft Gelatin capsules 80 mg** | | | | |
| **Tablet No.** | 15mins | 30mins | 45mins | 60mins | Infinity mins |
| **1** | 29 | 82 | 100 | 101 | 101 |
| **2** | 34 | 86 | 101 | 102 | 102 |
| **3** | 32 | 79 | 97 | 99 | 99 |
| **4** | 36 | 86 | 101 | 101 | 101 |
| **5** | 37 | 81 | 99 | 101 | 101 |
| **6** | 32 | 83 | 99 | 100 | 100 |
| **Mean** | 33 | 83 | 100 | 101 | 101 |
| **Minimum** | 29 | 79 | 97 | 99 | 99 |
| **Maximum** | 37 | 86 | 100 | 101 | 101 |
| **%RSD** | 8.8 | 3.4 | 1.5 | 1.0 | 1.0 |

The above experimental results reveal that 100% (w/w) of the drotaverine hydrochloride dissolved is released after 30 min for the NoSpa® tablet and after 45 min for a composition according to the invention.

### Pharmacokinetic simulation:

The pharmacokinetic blood profiles for the pharmaceutical composition of the present invention were calculated using a simulation program.
A mathematical model (Gastroplus™ simulator software available from Simulations Plus, Incorporated) was developed to explore the input rates for drotaverine dosage forms that would meet certain in vivo release targets. Gastroplus® is a computer program that simulates absorption and pharmacokinetics for orally dosed drugs. The underlying model is the Advanced Compartmental Absorption and Transit (ACAT) model - an extension of work originally done by Gordon Amidon and Lawrence Yu. See L. X. Yu, "An Integrated Model for Determining Causes of Poor Oral Drug Absorption." Pharm. Res.. 16:1883-7 (1999) and B. Agoram, W. S. Woltosz, and M. B. Bolger, "Predicting the impact of physiological and biochemical processes on oral drug bioavailability," Advanced Drug Delivery Reviews, 50:S41 -S67 (2001).

Gastroplus® was used to simulate the absorption and pharmacokinetics of the reference and test formulations. The *in vitro* dissolution profiles of drotaverine hydrochloride formulations (previously described) were used as input functions to simulate the absorption and pharmacokinetics of the reference (NoSpa® 80 mg commercial tablet) and test formulations.

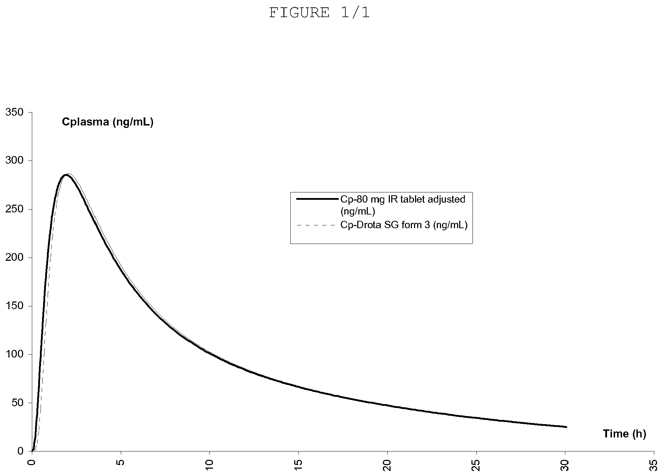

The figure 1 shows the results of using the model to simulate *in vivo* bioavailability of the NoSpa® tablet of 80 mg and of a pharmaceutical composition according to the invention containing 80 mg of drataverine hydrochloride.

The model was used to evaluate the performance of the dosage forms of the invention. These data show that the dosage forms of the invention will be effective. The AUC and the Cmax ratio were found to be comparable for a soft gel and tablet formulation of drotaverine hydrochloride at 80 mg using the Gastro Plus software.

## Claims

1. A pharmaceutical composition in the form of a soft capsule for oral administration comprising:
- 5 to 30% (w/w) of drotaverine hydrochloride;
- at least 60% (w/w) of a liquid mixture of a non-ionic hydrophilic surfactant and a non-ionic hydrophobic surfactant, wherein the non-ionic hydrophilic surfactant is polysorbate 80 and the non-ionic hydrophobic surfactant is propylene glycol monocaprylate;
- wherein the weight ratio of drotaverine hydrochloride to the liquid mixture of surfactant is from 1:3 to 1:7.

2. The pharmaceutical composition according to claim 1, wherein the total amount of surfactant is ranging from 75 to 90%.

3. The pharmaceutical composition according to claim 1, wherein the hydrophobic surfactant is present in amounts ranging from 60% to 90% by weight of the composition.

4. The pharmaceutical composition according to claim 1, wherein the non-ionic hydrophilic surfactant is present in amounts ranging from 3% to 7% by weight of the composition.

5. The pharmaceutical composition according to any one of claims 1 to 4, wherein the final pH value of the composition is between 4 and 6.

6. A method for preparing a pharmaceutical preparation according to any one of claims 1 to 5, comprising the following successive steps: dissolving the drotaverine hydrochloride in a liquid mixture of the non-ionic hydrophilic surfactant and the non-ionic hydrophobic surfactant, with stirring, in order to obtain an homogeneous mixture; and then adjust the pH between 4-6 using a conventional buffer.

7. A composition as claimed in any one of claims 1 to 5 for use in the treatment of spasms and acute pains.

## Patentansprüche

1. Pharmazeutische Zusammensetzung in der Form einer weichen Kapsel für die orale Verabreichung, umfassend:
- 5 bis 30 % (w/w) Drotaverinhydrochlorid;
- wenigstens 60 % (w/w) an einem flüssigen Gemisch eines nichtionischen hydrophilen grenzflächenaktiven Mittels und eines nichtionischen hydrophoben grenzflächenaktiven Mittels, wobei das nichtionische hydrophile grenzflächenaktive Mittel Polysorbat 80 ist und das nichtionische hydrophobe grenzflächenaktive Mittel Propylenglycolmonocaprylat ist;
- wobei das Gewichtsverhältnis von Drotaverinhydrochlorid zu dem flüssigen Gemisch von grenzflächenaktiven Mitteln von 1:3 bis 1:7 beträgt.

2. Pharmazeutische Zusammensetzung gemäß Anspruch 1, wobei die Gesamtmenge an grenzflächenaktivem Mittel in dem Bereich von 75 bis 90 % liegt.

3. Pharmazeutische Zusammensetzung gemäß Anspruch 1, wobei das hydrophobe grenzflächenaktive Mittel in Mengen in dem Bereich von 60 Gew.-% bis 90 Gew.-% der Zusammensetzung enthalten ist.

4. Pharmazeutische Zusammensetzung gemäß Anspruch 1, wobei das nichtionische hydrophile grenzflächenaktive Mittel in Mengen in dem Bereich von 3 Gew.-% bis 7 Gew.-% der Zusammensetzung enthalten ist.

5. Pharmazeutische Zusammensetzung gemäß einem der Ansprüche 1 bis 4, wobei der End-pH-Wert der Zusammensetzung zwischen 4 und 6 beträgt.

6. Verfahren zum Herstellen einer pharmazeutischen Zubereitung gemäß einem der Ansprüche 1 bis 5, umfassend folgende aufeinanderfolgende Schritte: Lösen des Drotaverinhydrochlorids in einem flüssigen Gemisch des nichtionischen hydrophilen grenzflächenaktiven Mittels und des nichtionischen hydrophoben grenzflächenaktiven Mittels unter Rühren, um ein homogenes Gemisch zu erhalten; dann Einstellen des pH-Werts auf zwischen 4 und 6 unter Verwendung eines herkömmlichen Puffers.

7. Zusammensetzung gemäß einem der Ansprüche 1 bis 5 für die Verwendung bei der Behandlung von Spasmen und akutem Schmerz.

## Revendications

1. Composition pharmaceutique sous la forme d'une capsule molle pour administration orale comprenant :
- 5 à 30 % en masse de chlorhydrate de drotavérine ;
- au moins 60 % en masse d'un mélange liquide d'un tensioactif hydrophile non ionique et d'un tensioactif hydrophobe non ionique, où le tensioactif hydrophile non ionique est le polysorbate 80 et le tensioactif hydrophobe non ionique est le monocaprylate de propylène glycol ;
- où le rapport massique de chlorhydrate de drotavérine sur le mélange liquide de tensioactif est compris entre 1:3 et 1:7.

2. Composition pharmaceutique selon la revendication 1, où la quantité totale de tensioactif est comprise entre 75 et 90 %.

3. Composition pharmaceutique selon la revendication 1, où le tensioactif hydrophobe est présent à des teneurs comprises entre 60 % et 90 % en masse de la composition.

4. Composition pharmaceutique selon la revendication 1, où le tensioactif hydrophile non ionique est présent à des teneurs comprises entre 3 % et 7 % en masse de la composition.

5. Composition pharmaceutique selon l'une quelconque des revendications 1 à 4, où la valeur de pH finale de la composition est comprise entre 4 et 6.

6. Procédé d'élaboration d'une préparation pharmaceutique selon l'une quelconque des revendications 1 à 5, comprenant les étapes successives suivantes : dissolution du chlorhydrate de drotavérine dans un mélange liquide du tensioactif hydrophile non ionique et du tensioactif hydrophobe non ionique, sous agitation, pour obtenir un mélange homogène ; puis ajustement du pH entre 4 et 6 en utilisant un tampon classique.

7. Composition selon l'une quelconque des revendications 1 à 5, pour utilisation dans le traitement des spasmes et des douleurs aiguës.
